# FASCICULE DE BREVET EUROPEEN

(11) **EP 1 819 664 B1**
(45) Date de publication et mention de la délivrance du brevet: **25.11.2009**
(21) Numéro de dépôt: 05822945.1
(22) Date de dépôt: 30.11.2005
(51) Int. Cl.: C07C 253/10, C07C 255/04

(54) **PROCEDE DE FABRICATION DE COMPOSES DINITRILES**
VERFAHREN ZUR HERSTELLUNG VON DINITRILEN
METHOD FOR THE PRODUCTION OF DINITRILES

(30) Priorité: 07.12.2004 FR 0412975
(43) Date de publication de la demande: 22.08.2007
(73) Titulaire: RHODIA CHIMIE, 93300 Aubervilliers (FR)
(72) Inventeur: LECONTE, Philippe, F-68150 Ribeauvillé (FR); BARATEAU, Béatrice, F-69230 Saint Genis Laval (FR)
(74) Mandataire: Esson, Jean-Pierre
(86) Numéro de dépôt international: PCT/FR2005/002978
(87) Numéro de publication internationale: WO 2006/061486

(56) Documents cités:
- DE-A1- 10 046 025
- US-A- 4 434 316
- US-A1- 2004 122 251
- US-B1- 6 197 992

## Description

La présente invention concerne un procédé de fabrication de composés dinitriles par double hydrocyanation d'une oléfine.
Elle concerne particulièrement un procédé de fabrication de composés dinitriles par double hydrocyanation d'une oléfine contenue dans un mélange d'hydrocarbures telle qu'une coupe pétrolière, et encore plus particulièrement une coupe pétrolière connue sous le nom de coupe C4.

Il est connu et utilisé industriellement un procédé de fabrication de dinitriles, notamment d'adiponitrile par double hydrocyanation d'une dioléfine telle que le 1,3 butadiène. Toutefois, pour éviter la formation de sous produits et la nécessité de prévoir de nombreuses étapes de séparation, le 1,3 butadiène utilisé doit contenir très peu d'impuretés.
Ce butadiène est extrait d'une coupe pétrolière appelée coupe C4 par un procédé de distillation extractive.

Pour éviter ces étapes de séparation et purification du butadiène, il a été proposé d'utiliser directement la coupe C4, donc un mélange d'hydrocarbures contenant des dioléfines telles que le butadiène, des alcènes notamment des butènes et des alcynes.

Ainsi, selon le brevet US 6 197 992, l'utilisation d'un tel mélange d'hydrocarbures est possible après avoir transformé dans le mélange les alcynes et les 1,2 diènes ou allènes par une hydrogénation sélective mise en oeuvre sur le mélange d'hydrocarbures.

En outre, le brevet US 4 434 316 décrit un procédé d'hydrocyanation d'un mélange d'hydrocarbures contenant des dioléfines et des alcènes. Le procédé permet de séparer et récupérer les alcènes qui réagissent plus lentement avec l'acide cyanhydrique. Toutefois, ce document ne décrit pas le procédé de récupération des produits comprenant une fonction nitrile.

Enfin, de nombreux brevets décrivent différents procédés de traitement et purification de la coupe C4, notamment des procédés d'hydrogénation sélective pour transformer les alcynes ou les allènes en alcanes ou alcènes non gênants dans le procédé d'hydrocyanation, au moins d'un point de vue chimique.

DE 100 46 025 divulgue un procédé dans lequel le dinitrile de l'acide adipique est préparé à partir de 1,3 butadiène et de l'acide cyanhydrique. Dans le cas dans ou des coupes C4 sont utilisées, le mélange est partiellement hydrogéné pour éliminer les alcynes.

Un des buts de la présente invention est de proposer un procédé de fabrication de composés dinitriles par double hydrocyanation d'un mélange d'hydrocarbures, sans traitement préalable, notamment un mélange d'hydrocarbures contenant des dioléfines et des composés tels que des alcynes.

A cet effet, l'invention propose un procédé de fabrication de composés dinitriles par double hydrocyanation d'un mélange d'hydrocarbures comprenant au moins une dioléfine et des alcynes caractérisé en ce qu'il consiste à:
- Réaliser une première hydrocyanation des dioléfines par réaction entre le mélange d'hydrocarbures et de l'acide cyanhydrique en présence d'un système catalytique comprenant un métal à l'état d'oxydation zéro et un ligand organophosphoré.
- A séparer du milieu réactionnel les nitriles insaturés linéaires pour former un premier flux PN1
- A réaliser sur ce premier flux PN1 une seconde hydrocyanation en présence d'acide cyanhydrique et un système catalytique comprenant un métal à l'état d'oxydation zéro, un ligand organophosphoré et un acide de Lewis
- A séparer, par distillation, au moins partiellement les nitriles insaturés non transformés pour former un second flux PN2.
- A séparer les composés dinitriles formés du système catalytique par une extraction liquide/liquide avec un solvant pour former un premier flux de dinitriles DN1
- alimenter conjointement les.dits flux PN2 et DN1 dans une étape de distillation pour produire une fraction de tête F1 contenant ledit solvant et une fraction de queue Q1 comprenant les composés dinitriles et nitrile insaturés.
- A alimenter la fraction de queue Q1 dans une deuxième étape de distillation pour séparer les nitriles insaturés formant une fraction de tête F2 des dinitriles formant une traction de queue Q2
- A alimenter la traction de queue Q2 dans une troisième étape de distillation pour obtenir une fraction contenant les dinitrile linéaires et une fraction de tête comprenant les composés de points d'ébullition plus faible à celui des dinitriles linéaire.
Par mélange d'hydrocarbures, on entend des mélanges contenant au moins une dioléfine permettant d'obtenir le dinitrile envisagé et d'autres hydrocarbures tels que, des alcynes, alcènes, allènes ou autres.

Généralement, ces mélanges d'hydrocarbures sont obtenus lors des opérations de raffinage du pétrole, sous forme de coupes appelées coupes pétrolières.
Dans le mode de réalisation préféré de l'invention, c'est-à-dire la préparation d'adiponitrile, la coupe pétrolière préférée est la coupe appelée C4 car contenant surtout des hydrocarbures comprenant 4 atomes de carbone, dont le 1,3 butadiène précurseur de l'adiponitrile.

Le procédé de double hydrocyanation d'une dioléfine consiste dans une première étape à réaliser l'addition d'un groupe cyano pour donner un mélange de mononitriles insaturés linéaires ou ramifiés tels que les pentènenitriles, les méthylbutènenitriles dans le cas de l'hydrocyanation du butadiène.

Parmi les pentènenitriles, les 3- et 4-pentènitriles sont les composés qui conduiront à l'adiponitrile dans une seconde étape d'hydrocyanation avec addition d'un second groupe cyano sur l'insaturation des mononitriles.
Ces réactions d'hydrocyanation sont mises en oeuvre en présence de système catalytiques homogènes ou hétérogènes.

Généralement, les systèmes catalytiques utilisés sont basés sur des complexes métalliques du nickel ou du palladium à l'état d'oxydation zéro avec des ligand organophosphorés tels que les organophosphites, organophosphinites, organophosphonites ou organophosphines mono ou pluridentates.
De tels ligands et systèmes catalytiques sont décrits dans de nombreux brevets, tels que, par exemple, les brevets français 1 544 656; 1 544 658; 1 589 943 ; 1 590 300, 1 590 300 ; 1 593 627 1 599 627 ; 2 196 326. Le procédé comprend en plus des deux étapes d'hydrocyanation une étape d'isomérisation réalisée en présence du système catalytique décrit ci-dessus, mais en absence d'acide cyanhydrique comme décrit dans le brevet français 1 589 943, par exemple. Dans cette étape le méthylbutènenitrile est isomérisé en 3- ou 4-pentènenitrile.

La deuxième étape d'hydrocyanation est généralement réalisée en présence d'un promoteur tel qu'un acide de Lewis. Le chlorure de zinc est l'acide de Lewis préférablement utilisé, au même titre que le triphénylborane.
Dans le procédé de fabrication des composés dinitriles par hydrocyanation d'une dioléfine, de nombreuses étapes de séparation et purification des différents produits sont mises en oeuvre.

Ainsi, en sortie du premier réacteur, la dioléfine n'ayant pas réagi et les hydrocarbures sont séparés par distillation ou flashage.
Les composés nitriles sont ensuite séparés du système catalytique par distillation ou flashage, le catalyseur étant recyclé au réacteur.

Avantageusement, le procédé comprend une séparation par distillation des nitriles insaturés linéaires et des nitriles insaturés ramifiés. Ces derniers sont introduits dans une étape d'isomérisation.

Les nitriles insaturés linéaires (flux PN1) sont introduits dans un second réacteur d'hydrocyanation. En sortie de ce second réacteur, les nitriles n'ayant pas réagi sont séparés, au moins partiellement, par distillation ou flashage pour former un flux de nitriles insaturés PN2.

Après cette séparation, les composés dinitriles sont séparés du système catalytique dans une étape d'extraction liquide/liquide avec un solvant type cyclohexane, comme par exemple décrit dans le brevet français 2 113 471. Le flux contenant les dinitriles appelés DN1 est engagé dans différentes étapes de purification pour extraire les résidus de solvant, les nitriles insaturés et enfin les dinitriles non linéaires et différents sous produits.
Le flux de nitriles PN2 est selon l'invention mélangé au flux DN1 à une de ces étapes de purification, de préférence, avant l'étape de séparation des composés nitriles insaturés et encore plus préférentiellement avant la colonne de séparation du solvant utilisé dans l'extraction liquide/liquide, comme indiqué ci-dessous.

Selon une caractéristique de l'invention, la purification du flux de dinitriles DN1 est effectuée, en premier lieu, dans une colonne permettant de séparer le solvant des composés nitriles, le flux PN2 de mononitriles étant alimenté conjointement au flux DN1 dans cette étape de séparation du solvant.
La fraction de queue Q1 contenant les composés dinitriles et nitriles insaturés est alors alimentée dans une seconde étape de distillation pour séparer les dinitriles des mononitriles insaturés. Ces derniers contenus dans la fraction de tête F2 sont avantageusement recyclés après séparation des mononitriles insaturés conjugués au niveau du second réacteur d'hydrocyanation.

Dans un mode de réalisation de l'invention, la séparation du solvant et des mononitriles insaturés peut être réalisée dans une seule colonne, à la place des deux colonnes comme décrit précédemment.

Enfin, les dinitriles linéaires tels que l'adiponitrile formant la fraction de queue Q2 sont récupérés dans une étape de distillation pour séparer les dinitriles linéaires des dinitriles ramifiés tels que le méthylglutaronitrile.

Le procédé de l'invention permet d'utiliser comme source de dioléfines, un mélange d'hydrocarbures contenant des composés alcynes. En effet, ces composés mis en présence du système catalytique d'hydrocyanation peuvent se trimériser. Ainsi, dans le cas d'une coupe C4 qui contient du propyne ou du butyne, ceux-ci, par trimérisation vont produire du triméthylbenzène (TMB) et du triéthylbenzène (TEB). Ces composés ont des points d'ébullition qui sont compris entre les nitriles insaturés et les dinitriles.
Selon le procédé de l'invention, comme les mononitriles du flux PN2 sont mélangés au flux DN1, il est possible de récupérer, en vue de leur recyclage dans le procédé d'hydrocyanation, les pentènenitriles exempts dé sous-produits de type TMB et/ou TEB car ceux-ci resteront dans la fraction contenant les dinitriles. En effet, dans la colonne de séparation entre les dinitriles et les mononitriles, les produits de trimérisation se trouveront dans la fraction de queue, c'est à dire dans la fraction contenant les dinitriles. Ainsi, les mononitriles insaturés qui sont recyclés au second réacteur d'hydrocyanation ne contiennent plus de produits de trimérisation. Ceux-ci seront purgés et séparés des composés dinitriles dans une des étapes de purification des dinitriles.
Le procédé de l'invention permet donc d'éliminer les produits de trimérisation en évitant leur accumulation et recyclage dans le second réacteur d'hydrocyanation.

Le procédé de l'invention permet donc d'utiliser comme matière première un mélange d'hydrocarbures contenant au moins une dioléfine et des alcynes.
Dans le cas de la fabrication d'adiponitrile, un intermédiaire chimique important pour la fabrication des monomères des polyamides tels que l'hexaméthylène diamine ou le caprolactame, le procédé de l'invention permet d'utiliser du butadiène non purifié, notamment contenant des alcynes. Ce résultat est important d'un point de vue économique car la séparation des alcynes et du butadiène requiert des étapes de distillation difficiles et coûteuses en investissement.

Le procédé de l'invention peut comprendre d'autres étapes permettant notamment de séparer le catalyseur ou d'extraire les restes de catalyseur présents sous forme solide tel que l'élément métallique à l'état oxydé.

Le procédé de l'invention permet donc d'utiliser comme matières premières pour la fabrication de l'adiponitrile, des mélanges d'hydrocarbures contenant du butadiène et notamment la coupe pétrolière C4 produite industriellement. Cette coupe C4 peut être utilisée directement sans traitement préalable ou après purification partielle. Toutefois, il n'est pas nécessaire de mettre en oeuvre une purification pour extraire les composés alcynes de cette coupe.

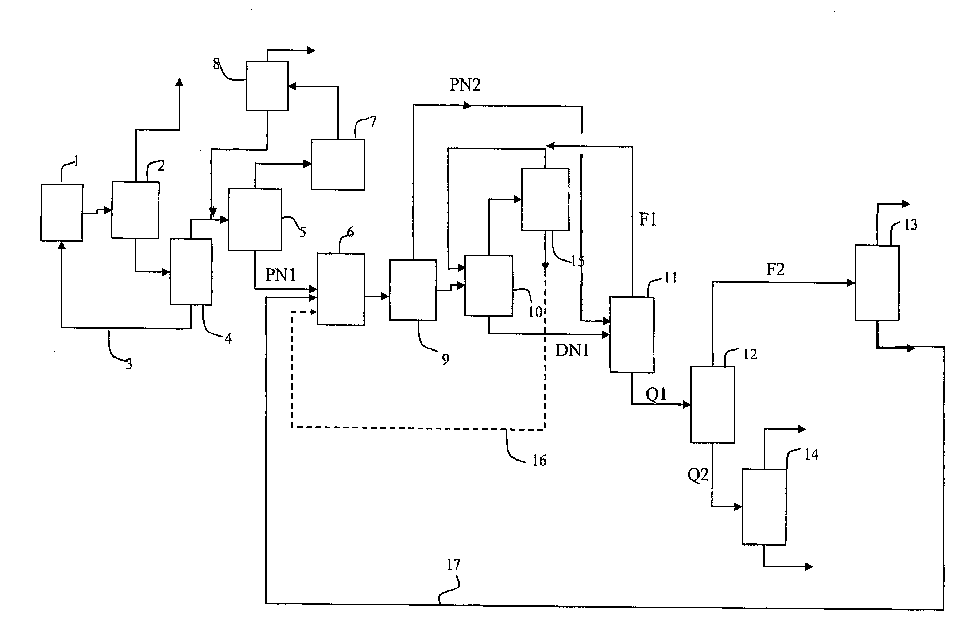

D'autres avantages, détails de l'invention apparaîtront clairement au vu d'un mode de réalisation du procédé de l'invention faite en référence à la figure unique annexée représentant un schéma synoptique du procédé de l'invention.

Selon un mode de réalisation de l'invention, un mélange d'hydrocarbures constitué pour une coupe C4 pétrolière est alimenté dans un réacteur 1 d'hydrocyanation avec de l'acide cyanhydrique. Le réacteur est un réacteur classique pour la mise en oeuvre, de réaction sous pression à haute température. Le système catalytique utilisé est, dans l'exemple de l'invention, un complexe de nickel à l'état d'oxydation zéro avec du triphénylphosphite.

Les conditions de cette réaction sont décrites dans la littérature, par exemple, dans les brevets cités précédemment et les brevets EP 1 344 770, US 5,981,772.

Le milieu réactionnel est soutiré du réacteur 1, et les hydrocarbures non transformés sont séparés dans une colonne 2.

Le flux contenant les composés nitriles insaturés est alimenté dans une colonne de flashage 4 permettant de séparer, en pied, le système catalytique, qui est recyclé dans le réacteur 1 par le conduit 3, et, en tête, les composés organiques, notamment, les composés nitriles insaturés.

Le flux contenant les composés mononitriles est avantageusement alimenté dans une colonne 5 de distillation pour récupérer en fraction de tête les nitriles insaturés ramifiés tels que le 2-méthyl-3-butène nitrile (2M3BN) et comme fraction de queue, les composés nitriles insaturés linéaires tels que les 3- pentènenitrile, 4- pentènenitriles, formant le flux PN1.

Ce dernier flux est alimenté dans le second réacteur d'hydrocyanation 6.
Le flux contenant les nitriles ramifiés est alimenté dans un réacteur 7 d'isomérisation. Cette réaction d'isomérisation est mise en oeuvre selon les conditions décrites, par exemples, dans les brevets US 5,981,772 et FR 1 589 943.
Le système catalytique utilisé dans cette étape d'isomérisation est avantageusement identique à celui utilisé dans le réacteur 1 de première étape d'hydrocyanation.
Le milieu réactionnel issu du réacteur 7 d'isomérisation est flashé pour séparer les composés organiques nitriles, du catalyseur. Les composés nitriles récupérés sont alimentés dans une étape de distillation 8 pour séparer, en tête, les composés nitriles conjugués ramifiés des composés nitriles non conjugués comme fraction de queue. Cette fraction de queue est renvoyée à l'alimentation de la colonne 5 de séparation des nitriles linéaires et nitriles ramifiés.

En sortie du second réacteur 6 d'hydrocyanation, le milieu réactionnel est soumis à une étape de distillation 9 ou flashage pour séparer les mononitriles non transformés recueillis comme fraction de tête et formant le flux PN2.

La fraction de queue contenant les dinitriles et le système catalytique est alimentée dans différents étapes de séparation dont une extraction liquide/liquide 10 pour séparer les dinitriles du système catalytique et récupérer ce dernier après séparation du solvant 15 pour recyclage par le conduit 16.

L'extraction liquide/liquide est mise en oeuvre en présence d'un solvant hydrocarbure permettant d'extraire le complexe organométallique. Comme solvant hydrocarbure convenable on peut citer le cyclohexane.

La phase contenant les dinitriles et un peu de solvant, formant le flux DN1, est, selon l'invention, mélangé avec le flux PN2 de mononitriles récupérés en sortie de l'étape de séparation 9.

Ce mélange est alimenté dans une colonne de distillation 11 pour séparer, en fraction de tête F1, les solvants résiduels tels que le cyclohexane.

La fraction de queue Q1 est alimentée dans une nouvelle colonne de distillation 12 pour récupérer en fraction de tête F2, les mononitriles et en fraction de queue Q2 les dinitriles.
Selon l'invention, les composés de trimérisation des alcynes tels que TMB ou TEB sont également récupérés dans la fraction de queue Q2 contenant les dinitriles.

Ainsi, la fraction de tête F2 contenant les mononitriles peut être recyclée au réacteur 6 de seconde hydrocyanation par le conduit 17 après avoir subi, une purification en 13 pour éliminer les mononitriles non valorisables en adiponitrile tels que le 2-pentènenitrile, valéronitrile.

Le flux Q2 contenant les dinitriles est ensuite alimenté dans une étape de purification 14 pouvant comprendre plusieurs colonnes de distillation montées en série permettant de récupérer une fraction comprenant les dinitriles ramifiés tels que le méthyl-3-glutaronitrile ainsi que les produits issus de la trimérisation des alcynes (TMB, TEB), et une fraction comprenant les dinitriles linéaires tels que l'adiponitrile.

Le procédé de l'invention permet ainsi d'éliminer comme effluents les produits de trimérisation des alcynes introduits avec le mélange d'hydrocarbures ou tout autre produit de point d'ébullition compris entre les nitriles et les dinitriles.

Un essai a été réalisé en alimentant dans la colonne de distillation 11 un mélange contenant de l'adiponitrile, du méthylglutaronitrile, du 3-pentènenitrile, du cyclohexane et du triméthyl benzène (TMB). La concentration pondérale du TMB est de 0,1 %. Ce mélange est alimenté selon un débit de 640 g/h.
La fraction de tête de la colonne 11 est constituée de cyclohexane et ne contient pas de TMB.
La fraction de queue contenant le TMB est alimentée dans la colonne 12 de séparation des mononitriles notamment du 3-pentènenitrile.
La fraction de tête récupérée à la colonne 12 est constituée principalement de 3-PN. La concentration pondérale en TMB est de 0,0005 %.
La fraction de queue comprenant les dinitriles est alimentée dans la colonne 14 de séparation et purification des dinitriles linéaires. La fraction de tête contenant notamment les dinitriles ramifiés, contient également du TMB. La quantité de TMB récupérée dans cette fraction de tête représente sensiblement la totalité du TMB engagé dans la colonne 11 (99,9 % de la quantité engagée).

Bien entendu, le procédé de l'invention peut comprendre d'autres étapes de séparation et purification des différents flux sans pour cela sortir du cadre de l'invention.

## Revendications

1. Procédé de fabrication de composés dinitriles par double hydrocyanation d'un mélange d'hydrocarbures comprenant au moins une dioléfine et des alcynes **caractérisé en ce qu'**il consiste à:
• Réaliser une première hydrocyanation des dioléfines par réaction entre le mélange d'hydrocarbures et de l'acide cyanhydrique en présence d'un système catalytique comprenant un métal à l'état d'oxydation zéro et un ligand organophosphoré.
• A séparer du milieu réactionnel les nitriles insaturés linéaires pour former un premier flux PN1
• A réaliser sur ce premier flux PN1 une seconde hydrocyanation en présence d'acide cyanhydrique et un système catalytique comprenant un métal à l'état d'oxydation zéro, un ligand organophosphoré et un acide de Lewis
• A séparer du milieu réactionnel, par distillation, les nitriles insaturés non transformés pour former un second flux PN2.
• A séparer du milieu réactionnel les composés dinitriles formés du système catalytique par une extraction liquide/liquide avec un solvant pour former un premier flux de dinitriles DN1
• A alimenter conjointement les dits flux PN2 et DN1 dans une étape de distillation pour produire une fraction de tête F1 contenant ledit solvant et une fraction de queue Q1 comprenant les composés dinitriles et nitriles insaturés.
• A alimenter la fraction de queue Q1 dans une deuxième étape de distillation pour séparer les nitriles insaturés formant une fraction de tête F2 des dinitriles formant une fraction de queue Q2
• A alimenter la fraction de queue Q2 dans une troisième étape de distillation pour obtenir une fraction contenant les dinitriles linéaires et une fraction de tête comprenant les composés de points d'ébullition plus faible à celui des dinitriles linéaires.

2. Procédé selon la revendication 1, **caractérisé en ce que** le mélange d'hydrocarbures comprend des dioléfines, plus particulièrement du butadiène.

3. Procédé selon la revendication 1 ou 2, **caractérisé en ce que** le mélange d'hydrocarbures est une coupe pétrolière C4.

4. Procédé selon l'une des revendications 1 à 3, **caractérisé en ce que** le système catalytique comprend un complexe du nickel à l'état d'oxydation zéro avec un composé organophosphoré.

5. Procédé selon l'une des revendications précédentes **caractérisé en ce que** le solvant utilisé dans l'étape d'extraction liquide/liquide est choisi dans le groupe comprenant le cyclohexane

6. Procédé selon l'une des revendications 1 à 5 **caractérisé en ce que** l'acide de Lewis est choisi dans le groupe comprenant le chlorure de zinc, le triphénylborane

## Claims

1. Process for the manufacture of dinitrile compounds by double hydrocyanation of a mixture of hydrocarbons comprising at least one diolefin and alkynes, **characterized in that** it consists:
• in carrying out a first hydrocyanation of the diolefins by reaction between the mixture of hydrocarbons and hydrocyanic acid in the presence of a catalytic system comprising a metal in the zero oxidation state and an organophosphorus ligand,
• in separating the linear unsaturated nitriles from the reaction medium in order to form a first stream PN1,
• in carrying out, on this first stream PN1, a second hydrocyanation in the presence of hydrocyanic acid and a catalytic system comprising a metal in the zero oxidation state, an organophosphorus ligand and a Lewis acid,
• in separating from the reaction medium, by distillation, the unconverted unsaturated nitriles in order to form a second stream PN2,
• in separating, from the reaction medium, the dinitrile compounds formed from the catalytic system by a liquid/liquid extraction with a solvent in order to form a first stream of dinitriles DN1,
• in jointly feeding the said streams PN2 and DN1 to a distillation stage in order to produce a top fraction F1 comprising the said solvent and a bottom fraction Q1 comprising the dinitrile and unsaturated nitrile compounds,
• in feeding the bottom fraction Q1 to a second distillation stage in order to separate the unsaturated nitriles, forming a top fraction F2, from the dinitriles, forming a bottom fraction Q2,
• in feeding the bottom fraction Q2 to a third distillation stage in order to obtain a fraction comprising the linear dinitriles and a top fraction comprising the compounds with lower boiling points than that of the linear dinitriles.

2. Process according to Claim 1, **characterized in that** the mixture of hydrocarbons comprises diolefins, more particularly butadiene.

3. Process according to Claim 1 or 2, **characterized in that** the mixture of hydrocarbons is a C4 petroleum fraction.

4. Process according to one of Claims 1 to 3, **characterized in that** the catalytic system comprises a complex of nickel in the zero oxidation state with an organophosphorus compound.

5. Process according to one of the preceding claims, **characterized in that** the solvent used in the liquid/liquid extraction stage is chosen from the group consisting of cyclohexane.

6. Process according to one of Claims 1 to 5, **characterized in that** the Lewis acid is chosen from the group consisting of zinc chloride and triphenylborane.

## Patentansprüche

1. Verfahren zur Herstellung von Dinitrilen durch doppelte Hydrocyanierung eines Kohlenwasserstoffgemischs, das mindestens ein Diolefin und Alkine enthält, **dadurch gekennzeichnet, daß** man:
• durch Reaktion zwischen dem Kohlenwasserstoffgemisch und Cyanwasserstoffsäure in Gegenwart eines Katalysatorsystems, das ein Metall in der Oxidationsstufe null und einen Organophosphorliganden enthält, eine erste Hydrocyanierung der Diolefine durchführt,
• die linearen ungesättigten Nitrile aus dem Rekationsmedium abtrennt, wobei man einen ersten Strom PN1 erhält,
• an diesem ersten Strom PN1 eine zweite Hydrocyanierung in Gegenwart von Cyanwasserstoffsäure und eines Katalysatorsystems, das ein Metall in der Oxidationsstufe null, einen Organophosphorliganden und eine Lewis-Säure enthält, durchführt,
• die nicht umgesetzten ungesättigten Nitrile durch Destillation aus dem Rekationsmedium abtrennt, wobei man einen zweiten Strom PN2 erhält,
• die von dem Katalysatorsystem gebildeten Dinitrilverbindungen durch Flüssig/Flüssig-Extraktion mit einem Lösungsmittel aus dem Reaktionsmedium abtrennt, wobei man einen ersten Dinitrilstrom DN1 erhält,
• die Ströme PN2 und DN1 zusammen einem Destillationsschritt zuführt, wobei man eine Kopffraktion F1, die das Lösungsmittel enthält, und eine Sumpffraktion Q1, die die Dinitrilverbindungen und ungesättigten Nitrilverbindungen enthält, erhält,
• die Sumpffraktion Q1 einem zweiten Destillationsschritt zuführt, wobei die ungesättigten Nitrile unter Bildung einer Kopffraktion F2 und einer Sumpffraktion Q2 getrennt werden,
• die Sumpffraktion Q2 einem dritten Destillationsschritt zuführt, wobei man eine Fraktion, die die linearen Dinitrile enthält, und eine Kopffraktion, die die Verbindungen mit niedrigeren Siedepunkten als die linearen Dinitrile enthält, erhält.

2. Verfahren nach Anspruch 1, **dadurch gekennzeichnet, daß** das Kohlenwasserstoffgemisch Diolefine, insbesondere Butadien, enthält.

3. Verfahren nach Anspruch 1 oder 2, **dadurch gekennzeichnet, daß** es sich bei dem Kohlenwasserstoffgemisch um einen C4-Erdölschnitt handelt.

4. Verfahren nach einem der Ansprüche 1 bis 3, **dadurch gekennzeichnet, daß** das Katalysatorsystem einen Komplex von Nickel in der Oxidationsstufe null mit einer Organophosphorverbindung umfaßt.

5. Verfahren nach einem der vorhergehenden Ansprüche, **dadurch gekennzeichnet, daß** man das bei dem Schritt der Flüssig/Flüssig-Extraktion verwendete Lösungsmittel aus der Gruppe bestehend aus Cyclohexan auswählt.

6. Verfahren nach einem der Ansprüche 1 bis 5, **dadurch gekennzeichnet, daß** man die Lewis-Säure aus der Gruppe bestehend aus Zinkchlorid und Triphenylboran auswählt.
